# EUROPEAN PATENT APPLICATION

(11) **EP 4 124 316 A1**
(43) Date of publication of application: **01.02.2023**
(21) Application number: 22187423.3
(22) Date of filing: 28.07.2022
(51) Int. Cl.: A61B 34/30, A61B 34/37

(54) **CASSETTE ASSEMBLY FOR ROBOTIC DRIVE**

(30) Priority: 30.07.2021 US 202163203795 P; 19.07.2022 US 202217813337
(71) Applicant: Corindus, Inc., Newton, MA 02466 (US)
(72) Inventor: BOUCHER, Wayne, Manchester, NH, 03109 (US); FALB, Peter, Hingham, MA, 02043 (US); PIAZZAROLO, Bruno, Waltham, MA, 02451 (US); CLARK, Andrew, Waltham, 02453 (US); GREGORY, Paul, Watertown, MA, 02472 (US)
(74) Representative: Horn Kleimann Waitzhofer Patentanwälte PartG mbB

(57) **Abstract**

A robotic drive system for driving one or more elongated medical devices includes a robotic drive including a first drive module; and a second drive module proximal to the first drive module each drive module independently moved along a longitudinal axis of the robotic drive. A sterile cassette assembly includes a first cassette; and a second cassette coupled to the first cassette with a coupler. The first cassette and the second cassette are removably attached together to the first drive module and the second drive module respectively.

## Description

### CROSS-REFERENCE TO RELATED PATENT APPLICATIONS

This application claims benefit of US Provisional Application No. 63/203,795 filed on July 30, 2021, entitled CASSETTE ASSEMBLY FOR ROBOTIC DRIVE, which is incorporated herein by reference in its entirety.

### FIELD

The present invention relates generally to the field of robotic medical procedure systems and, in particular, to a cassette assembly for a robotic drive.

### BACKGROUND

Catheters and other elongated medical devices (EMDs) may be used for minimally-invasive medical procedures for the diagnosis and treatment of diseases of various vascular systems, including neurovascular intervention (NVI) also known as neurointerventional surgery, percutaneous coronary intervention (PCI) and peripheral vascular intervention (PVI). These procedures typically involve navigating a guidewire through the vasculature, and via the guidewire advancing a catheter to deliver therapy. The catheterization procedure starts by gaining access into the appropriate vessel, such as an artery or vein, with an introducer sheath using standard percutaneous techniques. Through the introducer sheath, a sheath or guide catheter is then advanced over a diagnostic guidewire to a primary location such as an internal carotid artery for NVI, a coronary ostium for PCI, or a superficial femoral artery for PVI. A guidewire suitable for the vasculature is then navigated through the sheath or guide catheter to a target location in the vasculature. In certain situations, such as in tortuous anatomy, a support catheter or microcatheter is inserted over the guidewire to assist in navigating the guidewire. The physician or operator may use an imaging system (e.g., fluoroscope) to obtain a cine with a contrast injection and select a fixed frame for use as a roadmap to navigate the guidewire or catheter to the target location, for example, a lesion. Contrast-enhanced images are also obtained while the physician delivers the guidewire or catheter so that the physician can verify that the device is moving along the correct path to the target location. While observing the anatomy using fluoroscopy, the physician manipulates the proximal end of the guidewire or catheter to direct the distal tip into the appropriate vessels toward the lesion or target anatomical location and avoid advancing into side branches.

Robotic catheter-based procedure systems have been developed that may be used to aid a physician in performing catheterization procedures such as, for example, NVI, PCI and PVI. Examples of NVI procedures include coil embolization of aneurysms, liquid embolization of arteriovenous malformations and mechanical thrombectomy of large vessel occlusions in the setting of acute ischemic stroke. In an NVI procedure, the physician uses a robotic system to gain target lesion access by controlling the manipulation of a neurovascular guidewire and microcatheter to deliver the therapy to restore normal blood flow. Target access is enabled by the sheath or guide catheter but may also require an intermediate catheter for more distal territory or to provide adequate support for the microcatheter and guidewire. The distal tip of a guidewire is navigated into, or past, the lesion depending on the type of lesion and treatment. For treating aneurysms, the microcatheter is advanced into the lesion and the guidewire is removed and several embolization coils are deployed into the aneurysm through the microcatheter and used to block blood flow into the aneurysm. For treating arteriovenous malformations, a liquid embolic is injected into the malformation via a microcatheter. Mechanical thrombectomy to treat vessel occlusions can be achieved either through aspiration and/or use of a stent retriever. Depending on the location of the clot, aspiration is either done through an aspiration catheter, or through a microcatheter for smaller arteries. Once the aspiration catheter is at the lesion, negative pressure is applied to remove the clot through the catheter. Alternatively, the clot can be removed by deploying a stent retriever through the microcatheter. Once the clot has integrated into the stent retriever, the clot is retrieved by retracting the stent retriever and microcatheter (or intermediate catheter) into the guide catheter.

In PCI, the physician uses a robotic system to gain lesion access by manipulating a coronary guidewire to deliver the therapy and restore normal blood flow. The access is enabled by seating a guide catheter in a coronary ostium. The distal tip of the guidewire is navigated past the lesion and, for complex anatomies, a microcatheter may be used to provide adequate support for the guidewire. The blood flow is restored by delivering and deploying a stent or balloon at the lesion. The lesion may need preparation prior to stenting, by either delivering a balloon for pre-dilation of the lesion, or by performing atherectomy using, for example, a laser or rotational atherectomy catheter and a balloon over the guidewire. Diagnostic imaging and physiological measurements may be performed to determine appropriate therapy by using imaging catheters or fractional flow reserve (FFR) measurements.

In PVI, the physician uses a robotic system to deliver the therapy and restore blood flow with techniques similar to NVI. The distal tip of the guidewire is navigated past the lesion and a microcatheter may be used to provide adequate support for the guidewire for complex anatomies. The blood flow is restored by delivering and deploying a stent or balloon to the lesion. As with PCI, lesion preparation and diagnostic imaging may be used as well.

When support at the distal end of a catheter or guidewire is needed, for example, to navigate tortuous or calcified vasculature, to reach distal anatomical locations, or to cross hard lesions, an over-the-wire (OTW) catheter or coaxial system is used. An OTW catheter has a lumen for the guidewire that extends the full length of the catheter. This provides a relatively stable system because the guidewire is supported along the whole length. This system, however, has some disadvantages, including higher friction, and longer overall length compared to rapid-exchange catheters (see below). Typically to remove or exchange an OTW catheter while maintaining the position of the indwelling guidewire, the exposed length (outside of the patient) of guidewire must be longer than the OTW catheter. A 300 cm long guidewire is typically sufficient for this purpose and is often referred to as an exchange length guidewire. Due to the length of the guidewire, two operators are needed to remove or exchange an OTW catheter. This becomes even more challenging if a triple coaxial, known in the art as a triaxial system, is used (quadruple coaxial catheters have also been known to be used). However, due to its stability, an OTW system is often used in NVI and PVI procedures. On the other hand, PCI procedures often use rapid exchange (or monorail) catheters. The guidewire lumen in a rapid exchange catheter runs only through a distal section of the catheter, called the monorail or rapid exchange (RX) section. With a RX system, the operator manipulates the interventional devices parallel to each other (as opposed to with an OTW system, in which the devices are manipulated in a serial configuration), and the exposed length of guidewire only needs to be slightly longer than the RX section of the catheter. A rapid exchange length guidewire is typically 180-200 cm long. Given the shorter length guidewire and monorail, RX catheters can be exchanged by a single operator. However, RX catheters are often inadequate when more distal support is needed.

### SUMMARY

In accordance with an embodiment, a robotic drive system for driving one or more elongated medical devices includes a drive system comprising a robotic drive comprising a first drive module; and a second drive module proximal to the first drive module. Each drive module independently moved along a longitudinal axis of the robotic drive. A sterile cassette assembly comprising a first cassette and a second cassette coupled to the first cassette with a coupler. The first cassette and the second cassette are removably attached together to the first drive module and the second drive module respectively.

In one implementation the coupler comprises a first arm connected to the first cassette and a second arm connected to the second cassette, the second arm being slidably connected to the first arm.

In one implementation the first arm and the second arm are coupled allowing relative movement between the first arm and the second arm only along their respective longitudinal axes.

In one implementation the robotic drive system further includes a first flexible support having a first distal end and a first proximal end, the first distal end being removably secured to the first cassette and the first proximal end being secured to a proximal end of the first arm, wherein a portion of the first flexible support, intermediate the first distal end and the first proximal end, is positioned within the second cassette.

In one implementation the sterile cassette assembly further comprising a third cassette and wherein the coupler further comprises a third arm being slidably connected to the second arm, the third cassette connected to the third arm.

In one implementation the second arm comprises a first portion slidably engaged with the first arm and a second portion slidably engaged with the third arm.

In one implementation the sterile cassette assembly further comprises a fourth cassette and wherein the coupler further comprises having a fourth arm being slidably connected to the third arm, the fourth cassette connected to the fourth arm.

In one implementation the robotic drive further includes a second flexible support having a second distal end and second proximal end, the second distal end of the second flexible support being removably secured to the second cassette and the second proximal end being secured to a proximal end of the second arm, wherein a portion of the second flexible support intermediate the second distal end and the second proximal end is positioned within the third cassette.

In one implementation the robotic drive system further includes an initial flexible support having an initial distal end and an initial proximal end, the initial distal end being removably secured to a distal connector distal the first cassette and the initial proximal end being secured to a robotic drive housing, wherein a portion of the initial flexible support intermediate the initial distal end and the initial proximal end is positioned within the first cassette.

In one implementation the first arm comprises a first guide operatively guiding a portion of the initial flexible support between a distal sheath connector and a support anchor on a housing of the robotic drive.

In one implementation the second arm comprises a second guide operatively guiding a portion of the first flexible support between the first cassette and the proximal end of the first flexible support.

In one implementation the third arm includes a third guide operatively guiding a portion the second flexible support between the first second and the proximal end of the first flexible support.

In one implementation each cassette comprises a portion that rests on a surface of a corresponding drive module during attachment of each cassette to the corresponding drive module.

In one implementation the first cassette comprises a latch releasably engaging a tab in the drive module.

In one implementation the first cassette comprises a cylindrical cavity receiving a cylindrical member in the drive module.

In one embodiment a robotic drive system for driving one or more elongated medical devices comprises a robotic drive comprising a first drive module; a second drive module proximal to the first drive module and a third drive module proximal the second drive module, wherein each drive module moves independently along a longitudinal axis of the robotic drive. A sterile cassette assembly comprises a first cassette having a first arm; a second cassette having a second arm, and a third cassette having a third arm. A first flexible support having a distal end and a proximal end being attached to the first cassette, wherein a portion of the first flexible support extends through the second cassette. A second flexible support having a distal end and a proximal end being attached to the second cassette, wherein a portion of the second flexible support extends through the third cassette, The first cassette, second cassette and third cassette are movable independent of one another, and wherein the first arm, second arm and third arm are slidably connected to one another.

In one implementation the second arm comprises a second guide operatively guiding a portion of the first flexible support between the first cassette and a connector on the proximal end of the first arm.

In one implementation the robotic drive system further comprises an initial flexible support having a distal end secured to a sheath connector and a proximal end secured to a housing of the robotic drive, wherein a portion of the initial flexible support moves through a channel in the first cassette.

In one implementation the cassette assembly can balance on a portion of the drive modules in an unlocked position.

In one implementation each cassette comprises a latch that releasably locks each cassette to a corresponding drive module.

In one embodiment a sterile cassette assembly includes a first cassette, a second cassette, a third cassette, and a coupler coupling the first cassette, the second cassette, and the third cassette together. The first cassette, the second cassette and the third cassette are movable independent of one another toward and away from one another while coupled together with the coupler.

In one implementation the sterile cassette assembly includes a third cassette, wherein the coupler couples the first cassette, the second cassette, and the third cassette together, wherein the first cassette, the second cassette and the third cassette are movable independent of one another toward and away from one another while coupled together with the coupler.

In one implementation the coupler includes a first arm secured to the first cassette, a second arm secured to the second cassette, and a third arm secured to the third cassette. The first arm, second arm and third arm are slidably connected to one another.

In one implementation the sterile cassette assembly further includes a first flexible support having a distal end and a proximal end being attached to the first cassette, wherein a portion of the first flexible support extends through the second cassette; and a second flexible support having a distal end and a proximal end attached to the second cassette, wherein a portion of the second flexible support extends through the third cassette.

### BRIEF DESCRIPTION OF THE DRAWINGS

The invention will become more fully understood from the following detailed description, taken in conjunction with the accompanying drawings, wherein the reference numerals refer to like parts in which:
FIG. 1 is a perspective view of an exemplary catheter procedure system in accordance with an embodiment;
FIG. 2 is a schematic block diagram of an exemplary catheter procedure system in accordance with an embodiment.
FIG 3 is a perspective view of a cassette assembly.
FIG 4A is an exploded view of a cassette assembly.
FIG 4B is a perspective view of the cassette assembly in an expanded orientation.
FIG 4C is the cassette assembly in an install orientation.
FIG 5A is a side plan view of the robotic drive and cassette assembly in an expanded orientation.
FIG 5B is a side plan view of the robotic drive and cassette assembly in an expanded orientation with fluid lines extending in a downward direction.
FIG 6A is a close up view of a distal cassette of the cassette assembly taken generally along lines 6A-6A of FIG 5A.
FIG 6B is a close up view of a distal cassette of the cassette assembly taken generally along lines 6B-6B of FIG 5B.
FIG 7 is a partial plan of the cassette assembly taken generally along line 7-7 of FIG 5A.
FIG 8 is an exploded front perspective view of a cassette and corresponding drive module.
FIG 9 is an exploded rear perspective view of a cassette and corresponding drive module.
FIG 10 is a rear plan view of a cassette.
FIG 11 is a side cross sectional view a cassette taken generally along line 11-11 of FIG 10 attached to a drive module.
FIG 12 is a perspective view of a proximal portion of a cassette.
FIG 13 is a cross-sectional view of the distal portion of the cassette taken generally along line 13-13 of FIG 12.
FIG 14 is a close-up view of the drive member of a drive module.
FIG 15 is an isometric view of a robotic drive with drive modules and a cassette assembly.

### DETAILED DESCRIPTION OF THE EXAMPLE EMBODIMENTS

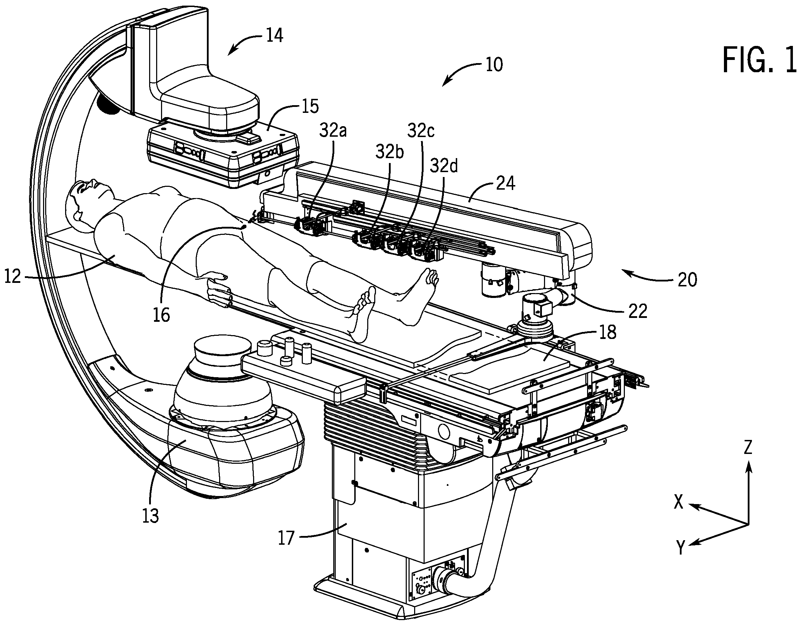

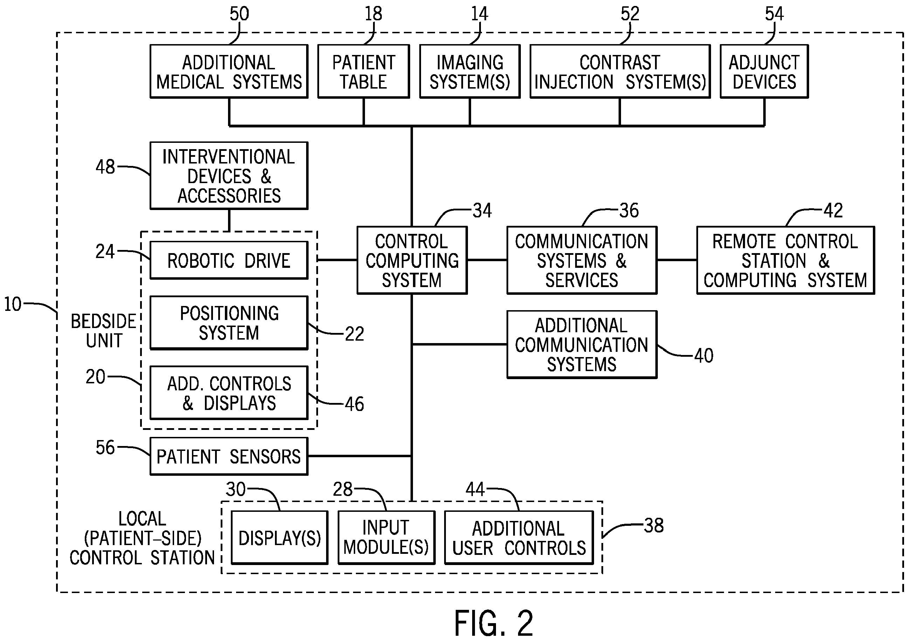

FIG. 1 is a perspective view of an example catheter-based procedure system 10 in accordance with an embodiment. Catheter-based procedure system 10 may be used to perform catheter-based medical procedures, e.g., percutaneous intervention procedures such as a percutaneous coronary intervention (PCI) (e.g., to treat STEMI), a neurovascular interventional procedure (NVI) (e.g., to treat an emergent large vessel occlusion (ELVO)), peripheral vascular intervention procedures (PVI) (e.g., for critical limb ischemia (CLI), etc.). Catheter-based medical procedures may include diagnostic catheterization procedures during which one or more catheters or other elongated medical devices (EMDs) are used to aid in the diagnosis of a patient's disease. For example, during one embodiment of a catheter-based diagnostic procedure, a contrast media is injected onto one or more arteries through a catheter and an image of the patient's vasculature is taken. Catheter-based medical procedures may also include catheter-based therapeutic procedures (e.g., angioplasty, stent placement, treatment of peripheral vascular disease, clot removal, arterial venous malformation therapy, treatment of aneurysm, etc.) during which a catheter (or other EMD) is used to treat a disease. Therapeutic procedures may be enhanced by the inclusion of adjunct devices 54 (shown in FIG. 2) such as, for example, intravascular ultrasound (IVUS), optical coherence tomography (OCT), fractional flow reserve (FFR), etc. It should be noted, however, that one skilled in the art would recognize that certain specific percutaneous intervention devices or components (e.g., type of guidewire, type of catheter, etc.) may be selected based on the type of procedure that is to be performed. Catheter-based procedure system 10 can perform any number of catheter-based medical procedures with minor adjustments to accommodate the specific percutaneous intervention devices to be used in the procedure.

Catheter-based procedure system 10 includes, among other elements, a bedside unit 20 and a control station (not shown). Bedside unit 20 includes a robotic drive 24 and a positioning system 22 that are located adjacent to a patient 12. Patient 12 is supported on a patient table 18. The positioning system 22 is used to position and support the robotic drive 24. The positioning system 22 may be, for example, a robotic arm, an articulated arm, a holder, etc. The positioning system 22 may be attached at one end to, for example, the patient table 18 (as shown in FIG. 1), a base, or a cart. The other end of the positioning system 22 is attached to the robotic drive 24. The positioning system 22 may be moved out of the way (along with the robotic drive 24) to allow for the patient 12 to be placed on the patient table 18. Once the patient 12 is positioned on the patient table 18, the positioning system 22 may be used to situate or position the robotic drive 24 relative to the patient 12 for the procedure. In an embodiment, patient table 18 is operably supported by a pedestal 17, which is secured to the floor and/or earth. Patient table 18 is able to move with multiple degrees of freedom, for example, roll, pitch, and yaw, relative to the pedestal 17. Bedside unit 20 may also include controls and displays 46 (shown in FIG. 2). For example, controls and displays may be located on a housing of the robotic drive 24.

Generally, the robotic drive 24 may be equipped with the appropriate percutaneous interventional devices and accessories 48 (shown in FIG. 2) (e.g., guidewires, various types of catheters including but not limited to balloon catheters, stent delivery systems, stent retrievers, embolization coils, liquid embolics, aspiration pumps, device to deliver contrast media, medicine, hemostasis valve adapters, syringes, stopcocks, inflation device, etc.) to allow a user or operator to perform a catheter-based medical procedure via a robotic system by operating various controls such as the controls and inputs located at the control station. Bedside unit 20, and in particular robotic drive 24, may include any number and/or combination of components to provide bedside unit 20 with the functionality described herein. The robotic drive 24 includes a plurality of device modules 32a-d mounted to a rail or linear member. Each of the device modules 32a-d may be used to drive an EMD such as a catheter or guidewire. For example, the robotic drive 24 may be used to automatically feed a guidewire into a diagnostic catheter and into a guide catheter in an artery of the patient 12. One or more devices, such as an EMD, enter the body (e.g., a vessel) of the patient 12 at an insertion point 16 via, for example, an introducer sheath. Each device module 32a-d include a drive module and cassette removably attached to the drive module.

Bedside unit 20 is in communication with the control station (not shown), allowing signals generated by the user inputs of the control station to be transmitted wirelessly or via hardwire to the bedside unit 20 to control various functions of bedside unit 20. As discussed below, control station 26 may include a control computing system 34 (shown in FIG. 2) or be coupled to the bedside unit 20 through the control computing system 34. Bedside unit 20 may also provide feedback signals (e.g., loads, speeds, operating conditions, warning signals, error codes, etc.) to the control station, control computing system 34 (shown in FIG. 2), or both. Communication between the control computing system 34 and various components of the catheter-based procedure system 10 may be provided via a communication link that may be a wireless connection, cable connections, or any other means capable of allowing communication to occur between components. The control station or other similar control system may be located either at a local site (e.g., local control station 38 shown in FIG. 2) or at a remote site (e.g., remote control station and computer system 42 shown in FIG. 2). Catheter procedure system 10 may be operated by a control station at the local site, a control station at a remote site, or both the local control station and the remote control station at the same time. At a local site, a user or operator and the control station are located in the same room or an adjacent room to the patient 12 and bedside unit 20. As used herein, a local site is the location of the bedside unit 20 and a patient 12 or subject (e.g., animal or cadaver) and the remote site is the location of a user or operator and a control station used to control the bedside unit 20 remotely. A control station (and a control computing system) at a remote site and the bedside unit 20 and/or a control computing system at a local site may be in communication using communication systems and services 36 (shown in FIG. 2), for example, through the Internet. In an embodiment, the remote site and the local (patient) site are away from one another, for example, in different rooms in the same building, different buildings in the same city, different cities, or other different locations where the remote site does not have physical access to the bedside unit 20 and/or patient 12 at the local site.

The control station generally includes one or more input modules 28 configured to receive user inputs to operate various components or systems of catheter-based procedure system 10. In the embodiment shown, control station allows the user or operator to control bedside unit 20 to perform a catheter-based medical procedure. For example, input modules 28 may be configured to cause bedside unit 20 to perform various tasks using percutaneous intervention devices (e.g., EMDs) interfaced with the robotic drive 24 (e.g., to advance, retract, or rotate a guidewire, advance, retract or rotate a catheter, inflate or deflate a balloon located on a catheter, position and/or deploy a stent, position and/or deploy a stent retriever, position and/or deploy a coil, inject contrast media into a catheter, inject liquid embolics into a catheter, inject medicine or saline into a catheter, aspirate on a catheter, or to perform any other function that may be performed as part of a catheter-based medical procedure). Robotic drive 24 includes various drive mechanisms to cause movement (e.g., axial and rotational movement) of the components of the bedside unit 20 including the percutaneous intervention devices.

In one embodiment, input modules 28 may include one or more touch screens, joysticks, scroll wheels, and/or buttons. In addition to input modules 28, the control station 26 may use additional user controls 44 (shown in FIG. 2) such as foot switches and microphones for voice commands, etc. Input modules 28 may be configured to advance, retract, or rotate various components and percutaneous intervention devices such as, for example, a guidewire, and one or more catheters or microcatheters. Buttons may include, for example, an emergency stop button, a multiplier button, device selection buttons and automated move buttons. When an emergency stop button is pushed, the power (e.g., electrical power) is shut off or removed to bedside unit 20. When in a speed control mode, a multiplier button acts to increase or decrease the speed at which the associated component is moved in response to a manipulation of input modules 28. When in a position control mode, a multiplier button changes the mapping between input distance and the output commanded distance. Device selection buttons allow the user or operator to select which of the percutaneous intervention devices loaded into the robotic drive 24 are controlled by input modules 28. Automated move buttons are used to enable algorithmic movements that the catheter-based procedure system 10 may perform on a percutaneous intervention device without direct command from the user or operator 11. In one embodiment, input modules 28 may include one or more controls or icons (not shown) displayed on a touch screen (that may or may not be part of a display), that, when activated, causes operation of a component of the catheter-based procedure system 10. Input modules 28 may also include a balloon or stent control that is configured to inflate or deflate a balloon and/or deploy a stent. Each of the input modules 28 may include one or more buttons, scroll wheels, joysticks, touch screen, etc. that may be used to control the particular component or components to which the control is dedicated. In addition, one or more touch screens may display one or more icons (not shown) related to various portions of input modules 28 or to various components of catheter-based procedure system 10.

Catheter-based procedure system 10 also includes an imaging system 14. Imaging system 14 may be any medical imaging system that may be used in conjunction with a catheter based medical procedure (e.g., non-digital X-ray, digital X-ray, CT, MRI, ultrasound, etc.). In an exemplary embodiment, imaging system 14 is a digital X-ray imaging device that is in communication with the control station. In one embodiment, imaging system 14 may include a C-arm (shown in FIG. 1) that allows imaging system 14 to partially or completely rotate around patient 12 in order to obtain images at different angular positions relative to patient 12 (e.g., sagittal views, caudal views, anterior-posterior views, etc.). In one embodiment imaging system 14 is a fluoroscopy system including a C-arm having an X-ray source 13 and a detector 15, also known as an image intensifier.

Imaging system 14 may be configured to take X-ray images of the appropriate area of patient 12 during a procedure. For example, imaging system 14 may be configured to take one or more X-ray images of the head to diagnose a neurovascular condition. Imaging system 14 may also be configured to take one or more X-ray images (e.g., real time images) during a catheter-based medical procedure to assist the user or operator 11 of control station 26 to properly position a guidewire, guide catheter, microcatheter, stent retriever, coil, stent, balloon, etc. during the procedure. The image or images may be displayed on display 30. For example, images may be displayed on a display to allow the user or operator to accurately move a guide catheter or guidewire into the proper position.

In order to clarify directions, a rectangular coordinate system is introduced with X, Y, and Z axes. The positive X axis is oriented in a longitudinal (axial) distal direction, that is, in the direction from the proximal end to the distal end, stated another way from the proximal to distal direction. The Y and Z axes are in a transverse plane to the X axis, with the positive Z axis oriented up, that is, in the direction opposite of gravity, and the Y axis is automatically determined by right-hand rule.

FIG. 2 is a block diagram of catheter-based procedure system 10 in accordance with an example embodiment. Catheter-procedure system 10 may include a control computing system 34. Control computing system 34 may physically be, for example, part of a control station. Control computing system 34 may generally be an electronic control unit suitable to provide catheter-based procedure system 10 with the various functionalities described herein. For example, control computing system 34 may be an embedded system, a dedicated circuit, a general-purpose system programmed with the functionality described herein, etc. Control computing system 34 is in communication with bedside unit 20, communications systems and services 36 (e.g., Internet, firewalls, cloud services, session managers, a hospital network, etc.), a local control station 38, additional communications systems 40 (e.g., a telepresence system), a remote control station and computing system 42, and patient sensors 56 (e.g., electrocardiogram (ECG) devices, electroencephalogram (EEG) devices, blood pressure monitors, temperature monitors, heart rate monitors, respiratory monitors, etc.). The control computing system is also in communication with imaging system 14, patient table 18, additional medical systems 50, contrast injection systems 52 and adjunct devices 54 (e.g., IVUS, OCT, FFR, etc.). The bedside unit 20 includes a robotic drive 24, a positioning system 22 and may include additional controls and displays 46. As mentioned above, the additional controls and displays may be located on a housing of the robotic drive 24. Interventional devices and accessories 48 (e.g., guidewires, catheters, etc.) interface to the bedside system 20. In an embodiment, interventional devices and accessories 48 may include specialized devices (e.g., IVUS catheter, OCT catheter, FFR wire, diagnostic catheter for contrast, etc.) which interface to their respective adjunct devices 54, namely, an IVUS system, an OCT system, and FFR system, etc.

In various embodiments, control computing system 34 is configured to generate control signals based on the user's interaction with input modules 28 (e.g., of a control station such as a local control station 38 or a remote control station 42) and/or based on information accessible to control computing system 34 such that a medical procedure may be performed using catheter-based procedure system 10. The local control station 38 includes one or more displays 30, one or more input modules 28, and additional user controls 44. The remote control station and computing system 42 may include similar components to the local control station 38. The remote 42 and local 38 control stations can be different and tailored based on their required functionalities. The additional user controls 44 may include, for example, one or more foot input controls. The foot input control may be configured to allow the user to select functions of the imaging system 14 such as turning on and off the X-ray and scrolling through different stored images. In another embodiment, a foot input device may be configured to allow the user to select which devices are mapped to scroll wheels included in input modules 28. Additional communication systems 40 (e.g., audio conference, video conference, telepresence, etc.) may be employed to help the operator interact with the patient, medical staff (e.g., angio-suite staff), and/or equipment in the vicinity of the bedside.

Catheter-based procedure system 10 may be connected or configured to include any other systems and/or devices not explicitly shown. For example, catheter-based procedure system 10 may include image processing engines, data storage and archive systems, automatic balloon and/or stent inflation systems, medicine injection systems, medicine tracking and/or logging systems, user logs, encryption systems, systems to restrict access or use of catheter-based procedure system 10, etc.

As mentioned, control computing system 34 is in communication with bedside unit 20 which includes a robotic drive 24, a positioning system 22 and may include additional controls and displays 46 and may provide control signals to the bedside unit 20 to control the operation of the motors and drive mechanisms used to drive the percutaneous intervention devices (e.g., guidewire, catheter, etc.). The various drive mechanisms may be provided as part of a robotic drive 24.

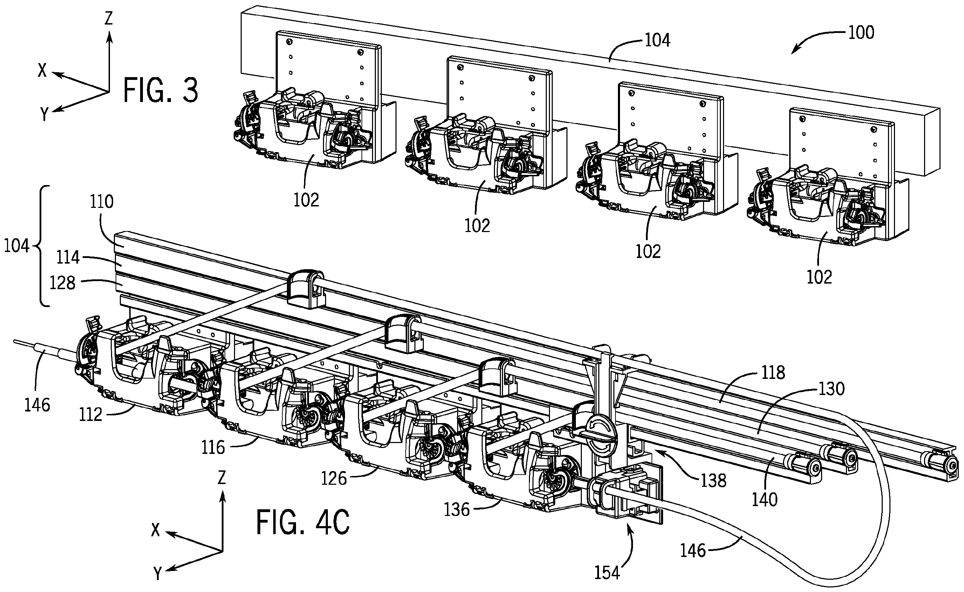

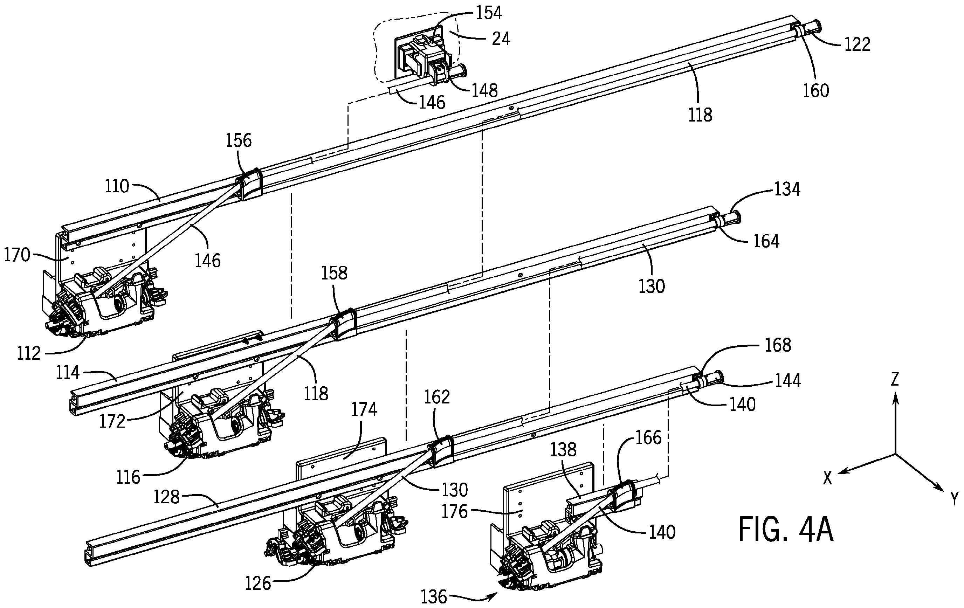

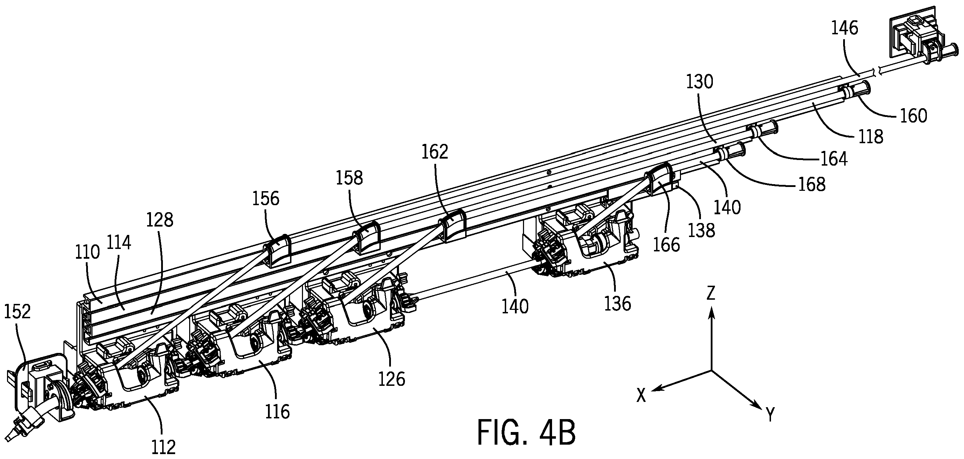

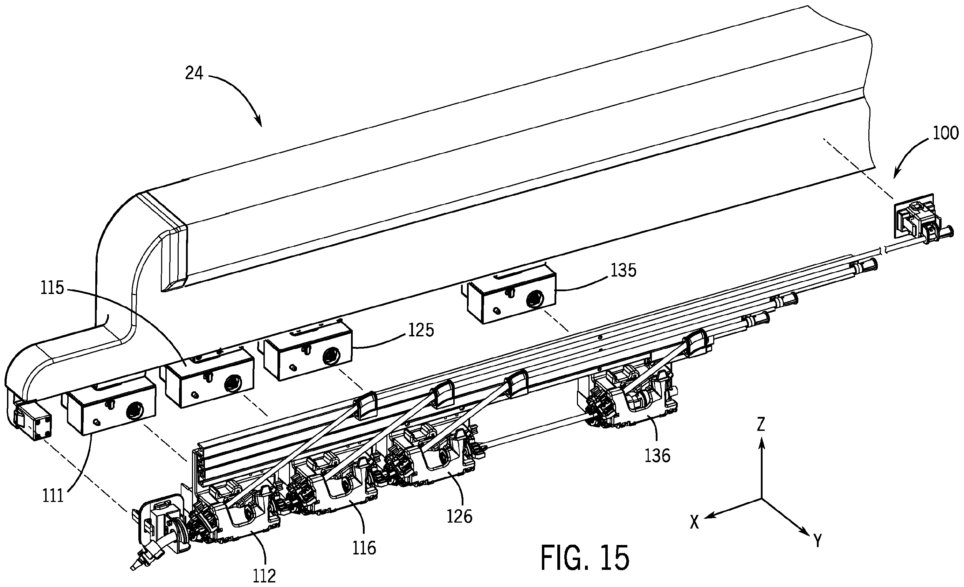

Referring to FIG 3 and FIG 15, a cassette assembly 100 includes a plurality of cassettes 102 coupled together with a coupler 104. The term cassette assembly as used herein includes an assembly of at least two cassettes. Referring to FIG 4A, 4B and 4C cassette assembly 100 is an assembly of at least a first cassette 112 and a second cassette 116. In one implementation robotic drive 24 includes the drive modules and a cassette assembly 100 is removably attached to the drive modules. The drive modules are part of the capital non-sterile portion of robotic drive 24. Cassette assembly 100 is part of the sterile portion of catheter-based procedure system 10. Each cassette 102 within the cassette assembly is attached to a respective drive module.

Attaching each cassette 102 to a respective drive module is required for each use of robotic drive 24. In procedures using multiple cassettes 102 cassette assembly 100 provides for a single loading step in which all cassettes 102 of cassette assembly 100 are loaded onto the respective drive modules. In one implementation coupler 104 is removably attached to cassettes 102 and is removed once each cassette has been attached to a respective drive module. The drive modules are moved to a loading configuration along the longitudinal axis of robotic drive 24. The drive modules are positioned relative to one another with predetermined spacing. The cassettes 102 of cassette assembly 100 are also positioned relative to one another with predetermined spacing in a loading position, such that each cassette 102 is aligned with a respective drive module during loading of cassette assembly 100 onto robotic drive 24.

In one implementation coupler 104 includes arms 108 that remain attached to the cassettes during operation of the catheter-based procedure system 10. Referring to FIG 4A, FIG 4B and FIG 4C coupler 104 includes a first arm 110 connected to the first cassette 112 and a second arm 114 connected to a second cassette 116, the second arm 114 being slidably connected to first arm 110. First arm 110 and second arm 114 each have a longitudinal axis. As discussed herein first arm 110 and second arm 114 are coupled to one another allowing relative movement between first arm 110 and second arm 114 only along their respective longitudinal axes. Other coupler devices that couple at least two cassettes and allow the at least two cassettes to move relative to one another while still being coupled are contemplated. In one implementation each first arm is connected to a respective cassette. Each arm may be formed integral with the cassette housing, or attached to the cassette housing as a separate component.

Cassette assembly 100 includes a first flexible support 118 having a first distal end 120 and a first proximal end 122. First distal end 120 is removably secured to first cassette 112 and first proximal end 122 is secured to a proximal 124 end first arm 110. A portion of first flexible support 118 intermediate first distal end 120 and first proximal end 122 is positioned within second cassette 116.

In one implementation cassette assembly 100 includes a third cassette 126 having a third arm 128. A second flexible support 130 has a second distal end 132 removably connected to a second proximal end of second cassette 116 and a second proximal end 134 secured to a proximal end of second arm 114. A portion of second flexible support 130 intermediate second distal end 132 and second proximal end 134 is positioned within third cassette 126.

In one implementation cassette assembly 100 includes a fourth cassette 136 having a fourth arm 138. A third flexible support 140 has a third distal end 142 removably connected to a proximal end of third cassette 126 and a third proximal end 144 secured to a proximal end of third arm 128. A portion of third flexible support 140 intermediate third distal end 142 and third proximal end 144 is positioned within third cassette 126.

In one implementation cassette assembly 100 includes an initial flexible support 146 having an initial proximal end 148 and an initial distal end 150. Initial distal end 150 is secured to a sheath connection 152 that connects initial flexible support 146 to an introducer sheath. Initial proximal end 148 is secured to a support anchor 154 that is attached to a housing of the robotic drive 24.

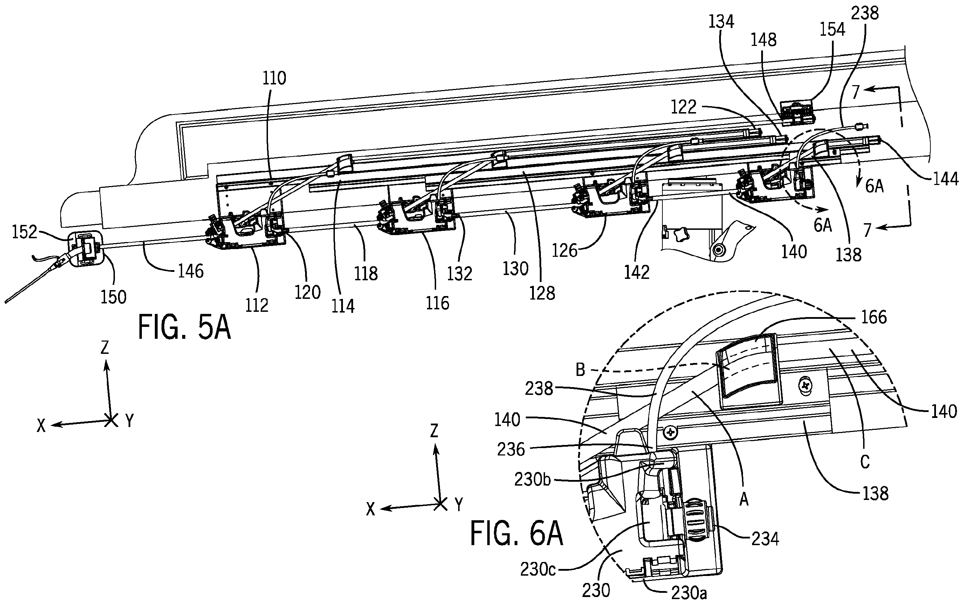

Referring to FIG 4A and FIG 5A in one implementation first arm 110 includes a first guide 156 that redirects the direction of the initial flexible support 146 toward support anchor 154 that connects initial proximal end 148 of flexible support 146 to the housing of robotic drive 24.

Second arm 114 includes a second guide 158 that redirects the direction of the first flexible support 118 toward a first connector 160 on a proximal portion of first arm 110.. First flexible support 118 includes a first distal end 120 and a first proximal end 122. First proximal end 122 is secured to a first connector 160 on first arm 110. First distal end 120 is removably secured to cassette 112.

Third arm 128 includes a third guide 162 that redirects the direction of the second flexible support 130 toward second connector 164 on a proximal portion of second arm 114. Second distal end 132 of second flexible support 130 is removably secured to second cassette 116. A second proximal end 134 of flexible support 130 is secured to second connector 164.

Fourth arm 138 includes a fourth guide 166 that redirects the direction of the third flexible support 140 toward a third connector 168 on a proximal portion of third arm 128. Third proximal end 144 of third flexible support 140 is secured to the third connector 168. Referring to FIG 6A fourth director 166 changes the direction of third flexible support 140 from a first orientation shown as region A through fourth director 166 shown as region B toward third connector 168 shown as region C.

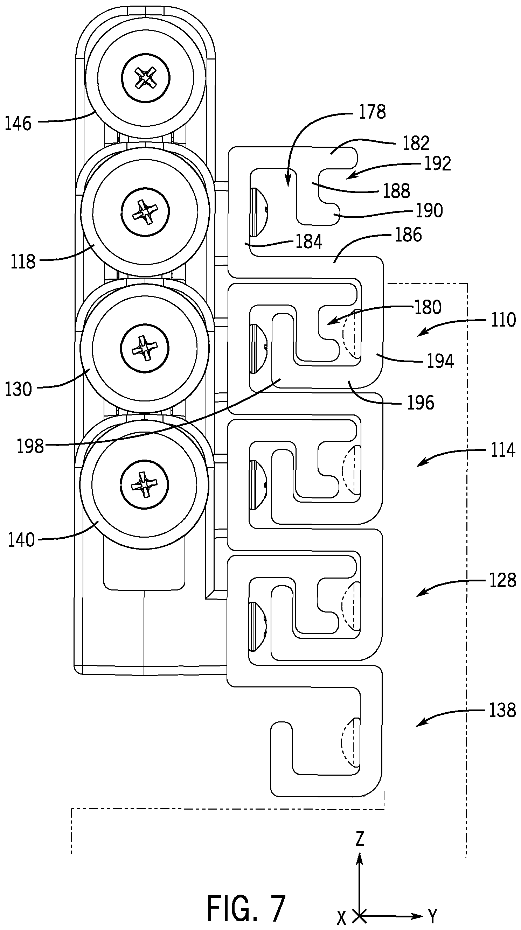

Referring to FIG 4A and FIG 7 first arm 110 is secured to a first cassette flange 170. In one implementation each first cassette 112, second arm 114, third cassette 126, and fourth cassette 136 include a respective first cassette flange 170, second cassette flange 172, third cassette flange 174, and fourth cassette flange 176. In one embodiment each flange is identical having four different connection zones. A first connection zone is furthest from the longitudinal axis of cassette assembly 100. In one embodiment first connection zone includes two apertures through which two fasteners connects first arm 110 to first flange 170. Similarly, a second connection zone second furthest from the longitudinal axis of cassette assembly 100 includes two apertures through which two fasteners connect second arm 114 to second cassette flange 172. A third connection zone being third furthest from the longitudinal axis of cassette assembly 100 includes two apertures through which two fasteners connect third arm 128 to third cassette flange 174. A fourth connection zone being closest to the longitudinal axis of cassette assembly 100 includes two apertures through which two fasteners connect fourth arm 138 to fourth cassette flange 176. The connection zones may include other fastener systems known in the art, such as other mechanical, chemical or material fastening systems.

In one implementation first arm 110, second arm 114, third arm 128 and fourth arm 138 are stacked one on top of another such that each includes a longitudinal axis spaced from and substantially parallel to the longitudinal axis of cassette assembly 100. Referring to FIG 5A each arm is stacked along the z' axis.

Referring to FIG 7, each of first arm 110, second arm 114, third arm 128 and fourth arm 138 have a similar cross section and may be formed from an extruded material. The cross section of first arm 110 has a general S-shape defining a first cavity 178 and a second cavity 180. First cavity is formed from a first wall 182, a second wall 184 and connecting wall 186. A guiding wall 188 extends from first wall 182 toward connecting wall 186 intermediate a free end of first wall 182 and second wall 184. Guiding wall 188 extends includes a tab 190 extending in a direction away from second wall 184 forming a pocket 192. Second cavity 180 is formed by connecting wall 186 a third wall 194 extending from connecting wall 186 in a direction away from first wall 182, a fourth wall 196 extends away from third wall, and a fifth wall 198 extending from fourth wall 196 in a direction toward connecting wall 186. Since each arm is identical each portion of each arm will be similarly identified.

Each arm is slidably coupled with an adjacent arm. Guiding wall and tab of second arm 114 is received within second cavity 180 of first arm 110 and fifth wall 198 of first arm 110 is positioned within the first cavity of second arm 114.

The guiding wall and tab of third arm 128 is received within the second cavity of second arm 114. Guiding wall and tab of fourth arm 138 is received within the second cavity of third arm 128. The interleaving of the arms minimizes movement of arms relative to one another in all directions except along the longitudinal axes of the arms.

Referring to FIG 4C cassette assembly 100 is show in a shipping configuration in which the proximal end of initial flexible support 146 and support anchor 154 are located adjacent fourth cassette 136.

Flexible supports in one implementation are tubes having a longitudinal slit extending substantially the entire length of the tube. Each cassette manipulates a percutaneous device that is supported between distal end of each cassette and the proximal cassette and/or sheath connector. The manner in which each percutaneous device enters and exits the lumen of the respective flexible supports is described in PCT Published application WO/2021/011551 entitled "SYSTEMS, APPARATUS AND METHODS FOR SUPPORTING AND DRIVING ELONGTED MEDICAL DEVICES IN A ROBOTIC CATHETER-BASED PROCEDURE SYSTEM" and incorporated herein by reference, in its entirety, to illustrate the manner in which flexible support tubes support percutaneous devices as multiple cassettes that move independently of one another and the manner in which a splitter within each cassette allows the percutaneous devices to enter the lumen of the flexible support tubes. The flexible supports allow for movement of the cassettes relative to one another without the percutaneous devices within the lumen of the flexible support tubes buckling. This is accomplished by the automatic adjustment of the length of flexible support between the cassettes. By way of example referring to FIG 5A as second cassette 116 moves in the proximal direction toward first cassette 112 a portion of first flexible support 118 moves through a channel in second cassette 116 toward first proximal end 122 of first arm 110. Note that second cassette 116 is secured to a drive module of robotic drive 24. Cassette 116 and its associated drive module form a device module that moves together. This is accomplished because first flexible support 118 is secured to a proximal end of the body of first cassette 112 at the proximal end of first flexible support 118 and secured to the proximal end of first arm 110 at the distal end of first flexible support 118. As second cassette 116 moves toward first cassette 112 first proximal end 122 connection is in tension so that first flexible support 118 does not buckle. Similarly, when second cassette 116 moves in the distal direction away from first cassette 112 the connection of the proximal end of first flexible support 118 places first flexible support 118 in tension. Note that the cassettes can move together at the same speed or different speeds and/or direction and the length of the flexible supports between the cassettes will be automatically adjusted.

Since first flexible support 118 is secured to the proximal end of the body of first cassette 112 and to the proximal end of first arm 110, flexible support 118 remains in tension as second cassette 116 moves relative to first cassette 112. As each cassette moves along the cassette assembly 100, the flexible support that extends through the respective cassette remains in tension since the flexible support is supported at both its distal end and proximal end. The distal end and proximal end locations are fixed distance relative to one another. In the example of the first flexible support 118, second flexible support 130, and third flexible support 140 the proximal and distal ends of each support is secured to the same cassette/arm arrangement. The proximal end and distal end of initial flexible support 146 is secured to sheath connection 152 and support anchor 154 respectively. Sheath connection 152 and support anchor 154 are in a fixed distance relationship in the install, in-use position, of catheter-based procedure system 10.

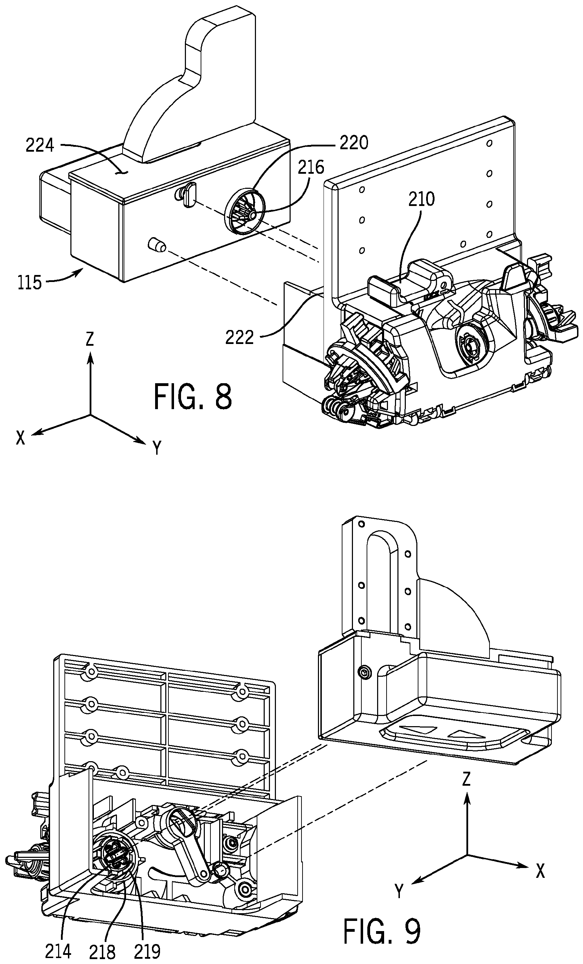

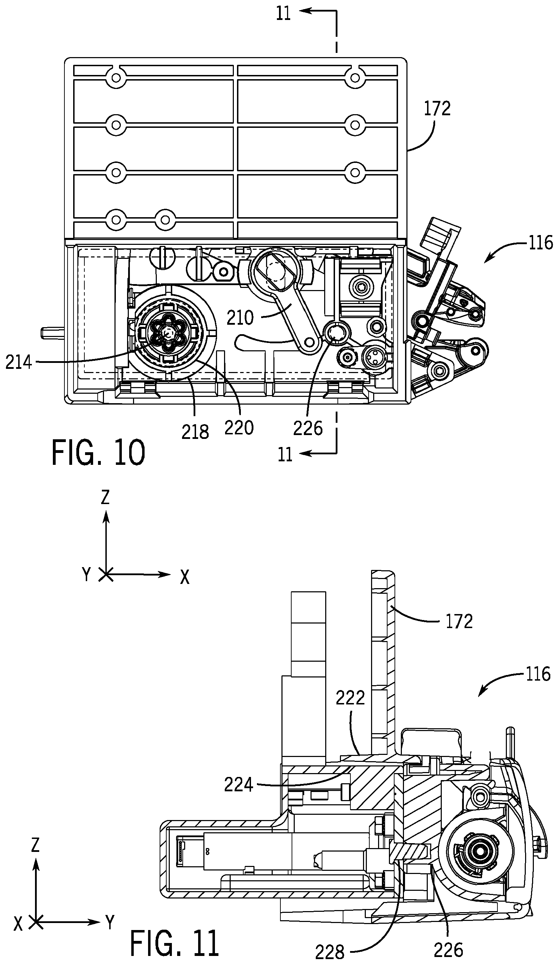

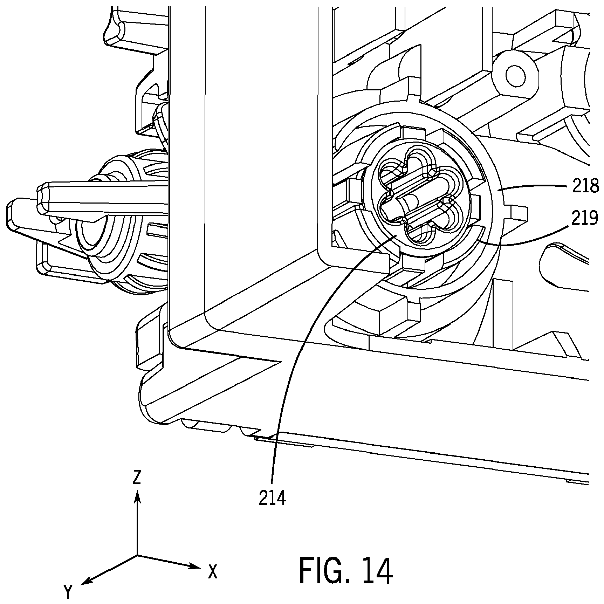

Referring to FIG 15 each of the first cassette 112, the second cassette 116, the third cassette 126, and the fourth cassette 136 is secured to a respective first drive module 111, a second drive module 115, a third drive module 125 and a fourth drive module 135 in a similar manner. Referring to FIG 8, FIG 9, FIG 10 , FIG 11 and FIG 14 in one implementation connection of each cassette to its respective drive module is the same. Accordingly, attachment of second cassette 116 to second drive module 115 is the same as attachment of the other cassettes their respective drive modules. Second cassette 116 includes a latch 210 that slidably moves from an unlocked position to a locked position. A portion of latch 210 releasably engages a tab on drive module 115. Second cassette 116 includes a driven member 214 that is releasably engaged with a drive member 216 of second drive module 115. Referring to FIG 14, driven member 214 is surrounded by an outer cylindrical wall 218 defining a cavity between driven member 214 and outer cylindrical wall 218. In one implementation the cavity is formed by outer cylindrical wall 218 and a second concentric cylindrical wall 219. Second drive module 115 includes an outwardly extending cylindrical wall 220 that is received within the cavity as cassette 116 is moved toward second drive module 115. The location of outwardly extending cylindrical wall 220 within the cavity assists in minimizing movement between first flexible support 118 and second drive module 115 in the installed and locked position. Driven member 214 is retained within drive module 115 by the resilient spring like portions of second concentric cylindrical wall 219. Stated another way second concentric cylindrical wall 219 includes a plurality of resilient finger like portions that have an upper lip at the free end of each finger portion that retains the driven member 214 within drive module 115.

First flexible support 118 includes a rest wall portion 222 that rests on a wall portion 224 on second drive module 115 when cassette assembly 100 is moved to a first install position. The rest wall portion of each cassette of cassette assembly 100 rests on all corresponding support wall portions of the respective drive modules. In one implementation, cassette assembly 100 is fully supported on the drive modules in this first install position, such that a person or operator installing cassette assembly 100 onto the robotic drive 24 need not hold cassette assembly 100. Stated another way, cassette assembly 100 is balanced on the drive modules allowing a user to release both hands from the cassette assembly prior to latching the cassettes to the drive modules. Each cassette includes a locating recess 226 that receives a locating pin 228. An operator moves cassette assembly 100, once resting on the support surfaces, in a direction toward the drive module 115 such that each tab is positioned within each recess and each cylindrical wall is within each cavity. In this engaged position, each latch is moved from the unlocked position to the locked position thereby locking each cassette to a respective drive module.

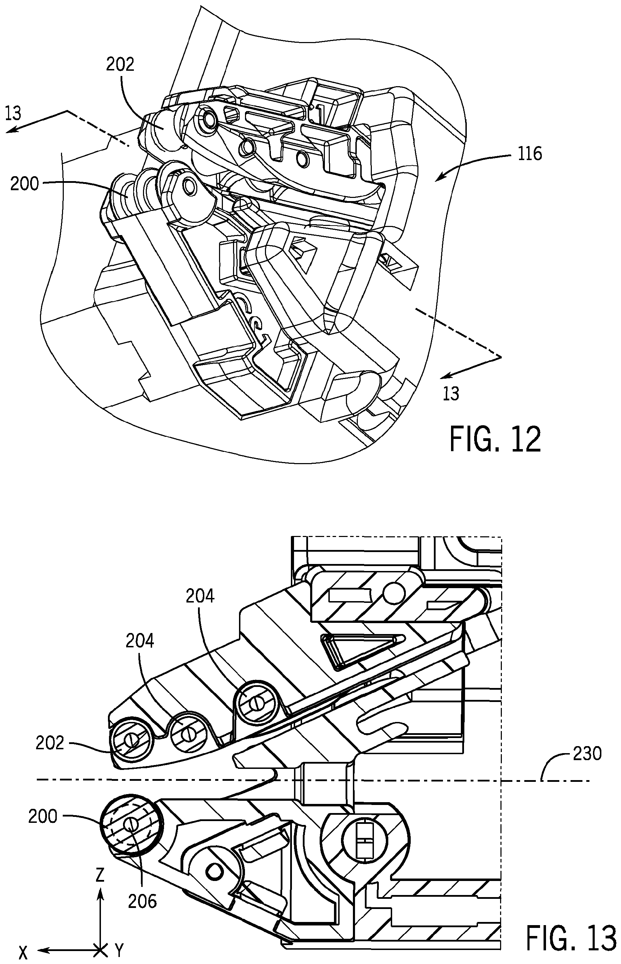

Referring to FIG 12 and FIG 13 the proximal end of the body of second cassette 116 includes a first roller 200 and a second roller 202 to help guide the portion of first flexible support 118 as it moves through the second cassette 116 about second guide 158 toward first connector 160. In one implementation, additional rollers 204 are provided to facilitate smooth movement of first flexible support 118 through second cassette 116. Roller 200 may pivot about a pin 206 as first flexible support 118 moves through second cassette 116.

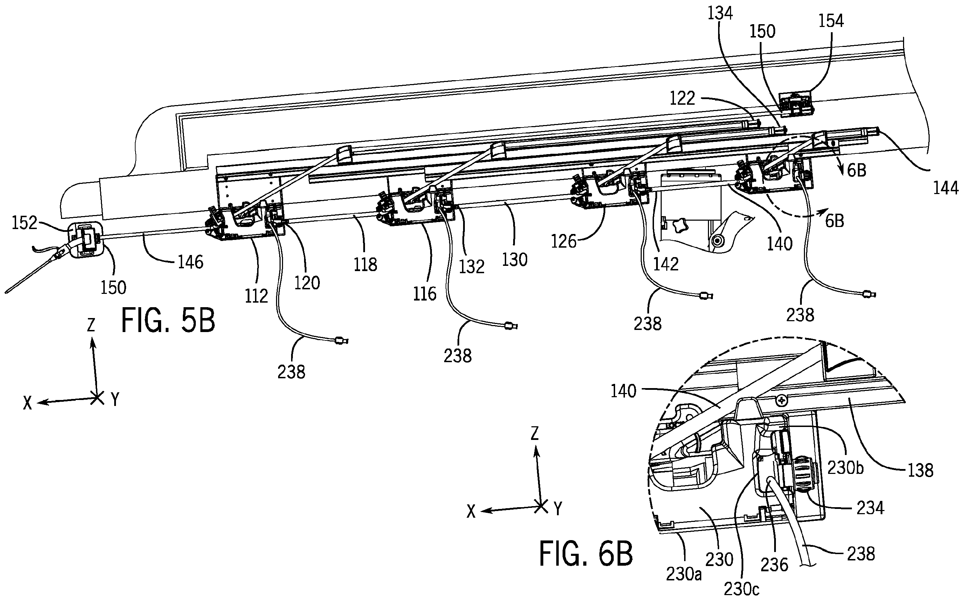

Referring to Referring to FIG 5A, FIG 5B, FIG 6A and FIG 6B, in one implementation, a fluid line 238 in fluid communication with hemostasis valve 234 through a port 236 as is known in the art. In one implementation fluid lines 238 that are connected to a port 236 of a respective hemostasis valve to deliver a fluid to the hemostasis valve such as contrast agent, saline, therapeutic, or other fluids known in the art. Fluid lines 238 are generally in the shape of a tube having a lumen extending therethrough. While FIG 6A and FIG 6B illustrate the fourth cassette and most proximal cassette in the cassette assembly includes a fluid line, in one implementation of the system, the fourth or most proximal cassette would include a wire-based device that would be coupled to the cassette with a collet but without a hemostasis valve. It is also contemplated that the most proximal device could include a catheter and a hemostasis valve. Note that the description of the fluid lines relative to the most proximal cassette the fluid lines are also applicable to the more distal cassettes. Similarly, it is contemplated that not all of the cassettes include a fluid line with a hemostasis valve. Stated another way in one implementation some but not all of the cassettes in the cassette assembly include a fluid line.

Referring to FIG 5A and FIG 6A in one implementation fluid line 238 extends from port 236 in a generally upward direction along the positive Z-axis. In this implementation the fluid lines 238 extend from hemostasis valve in a direction away from the patient table 18. A cover 230 is pivotally attached to each drive module proximate a bottom portion 230a of cover 230. A top portion 230b restrains a portion of fluid line 238 and directs fluid line 238 in an upwardly orientation along the positive Z-axis in a direction away from the patient table 18.

Referring to FIG 5B and FIG 6B in one implementation fluid line 238 extends from port 236 which extends in a first generally cross-table direction along the positive Y-axis and then the flexible fluid line 238 under the weight of gravity extends toward patient table 18. An opening 230c in the proximal side of cover 230 allows fluid line 238 to extend through cover 230. Opening 230c has a lower edge, an opposing upper edge and a distal edge. Opening 230c is completely open on the proximal edge. The lower edge and upper edge of opening 230c aids to guide the fluid line 238 as it extends from port 236. In one implementation, port 236 is a side port extending directly from the hemostasis valve body. However, it is also contemplated that fluid line 238 could be fluidly coupled to a leg of a Y-connector extending at an angle to the longitudinal axis of the hemostasis valve. In one implementation fluid line 238 is removably and fluidly coupled to a leg of a Y-connector extending at an angle to the longitudinal axis of the hemostasis valve. In one implementation the orientation of the side port with fluid line is at 90 degrees (parallel to patient table) and in one implementation the orientation of the side port with the fluid line is drooping towards the patient table. The open proximal side of cover opening 230c allows for fluid line 238 to easily positioned within opening 230c of cover 230 as cover 230 is pivoted about the bottom portion 230a to the closed position. Referring to FIG 4B and FIG 5B, all of the fluid lines 238 are away from arms 110, 114, 128 and 138 thereby avoiding entanglement of the fluid lines and associated coupling devices as are known in the art (e.g., stopcocks.) Stated another way all of the fluid lines 238 are directed away from or do not pass near arms 110, 114, 128 and 138. In one implementation (not shown), cover opening 230c does not have an upper edge thereby allowing fluid line 238 to positioned within cover 230 without the need to move the fluid line through the side opening on the proximal side of opening 230c. While certain commercially available hemostasis valves have a port 236 that can removably receive an end of a fluid lines other commercially available hemostasis valves have a fluid line integrated with and connected to the port 236 that are not readily removable. In one implementation a connector provides for fluid lines to be removed. One example of not readily removable fluid line from port 236 is where no connector is provided. Fluid lines that are removable and integrated are contemplated as alternative implementations. Where the fluid lines are not integrated, the cover can be pivoted from the open to closed position without encountering the side port of the hemostasis valve y-connector. Where the fluid line is integrated with port 236 it is advantageous to allow cover 230 to be pivoted from the fully open to fully closed position without encountering fluid line 238. In some procedures such as in a neurovascular intervention procedure, each fluid line is connected to tubing coming from a pressurized saline bag. The saline bags typically hang from poles mounted to the rail on the left side of the patient table. The fluid lines and tubing are then routed under the robotic drive and as such provides access for the bedside user to manipulate the stopcock or roller clamp (or associated coupling device. In one implementation cover 230 does not extend into the space where the side port protrudes. Such that cover 230 does not contact the fluid line as the cover is moved from an open to a closed position.

The term couple, coupled, coupler, coupling as used herein could be a flexible coupler, a rigid coupler, a slidable coupler, a releasable coupler, or other couplers that allow each separate cassette to be positioned together to be positioned relative to a respective drive module at the same time.

Hemostasis valves are known in the art. A conventional hemostasis valve has a rotating seal at the end , which is turned open or closed each time a wire or microcatheter/guidewire is introduced or extracted. Stated another way the seal itself in one implementation does not rotate, rather a touhy-borst valve is compressed by rotating the proximal end. Though not all hemostasis valves have touhy-borst valves. They are used to seal off and minimize fluid loss during interventional and diagnostic procedures. Hemostasis valves allow instruments such as catheters or other EMDs to open and pass through the valve and close automatically as soon as the instrument is withdrawn . Note A cross-cut elastomeric valve (or similar) will close when the EMD is withdrawn. However, not all hemostasis valves have a cross-cut elastomeric valve. A hemostasis valve includes a first leg having a distal end and a proximal end. A second leg extends from first leg and is in fluid communication with first leg such that a fluid may be introduced into a proximate end of second leg. Hemostasis valve first leg defines a longitudinal axis extending from proximal end of first leg to distal end of first leg. The distal end of first leg may include a luer connector that may be rotatably coupled to distal end of first leg. Rotating luer connector includes an external surface and an internal region having a luer female interface to releasably couple a guide catheter. Luer connectors are known in the art and provide a fluid tight connection between a guide catheter and a hemostasis valve. Luer connectors are covered by standard ISO 80369-7 . A rotating hemostasis valve (RHV) attaches to the proximal hub of a catheter and allows another device to be inserted while maintaining a seal. The RHV has a side port to connect to a syringe, heparinized saline line, contrast injection system, manual/pump aspiration or a manifold. The Luer connector at the distal end of the RHV rotates independently from the rest of the RHV so that the side port does not rotate when the catheter device is rotated.

In one implementation a hemostasis valve device has one or more valves within body with a lumen. The body has a proximal end and distal end. The hemostasis valve device connects to the proximal hub of the catheter. In one implementation the hemostasis valve device is removably connected to the proximal hub of the catheter. In one implementation the hemostasis valve device is bonded to the catheter device. The hemostasis valve device in one implementation where the catheter is designed to be rotated includes a rotating connector that seals fluids. An elongated medical device (EMD) passes through the hemostasis valve device. In one implementation the hemostasis valve device includes a side port between the valves and the distal end of the hemostasis valve device. In one implementation the side port is not integral to the hemostasis valve device. The side port could be a separate component attached to the catheter device distal to the hemostasis valve. In one implementation the valve can be an elastomer with cross-cut (or other) geometry to create an aperture that allows an EMD to be inserted and closes/seals (due to the properties of the material) when the EMD is removed. In one implementation the valve can be a touhy borst valve that when compressed closes around the EMD or seals when the EMD is removed. When not compressed the valve has an aperture that remains open. Compression of the touhy-borst valve is typically done be rotating the proximal end of the hemostasis valve clockwise (CW). To close the touhy-borst valve, typically the proximal end of the hemostasis valve is rotated counterclockwise (CCW).

Although the present disclosure has been described with reference to example embodiments, workers skilled in the art will recognize that changes may be made in form and detail without departing from the spirit and scope of the defined subject matter. For example, although different example embodiments may have been described as including one or more features providing one or more benefits, it is contemplated that the described features may be interchanged with one another or alternatively be combined with one another in the described example embodiments or in other alternative embodiments. Because the technology of the present disclosure is relatively complex, not all changes in the technology are foreseeable. The present disclosure described is manifestly intended to be as broad as possible. For example, unless specifically otherwise noted, the definitions reciting a single particular element also encompass a plurality of such particular elements.

## Claims

1. A robotic drive system for driving one or more elongated medical devices, the drive system comprising:
a robotic drive comprising;
a first drive module (111); and
a second drive module (115) proximal to the first drive module (111), each drive module independently moved along a longitudinal axis of the robotic drive; and
a sterile cassette assembly (100) comprising:
a first cassette (112); and
a second cassette (116) coupled to the first cassette (112) with a coupler (104);
wherein the first cassette (112) and the second cassette (116) are removably attached together to the first drive module (111) and the second drive module (115) respectively.

2. The robotic drive system of claim 1, wherein the coupler (104) comprises a first arm (110) connected to the first cassette (112) and a second arm (114) connected to the second cassette (116), the second arm (114) being slidably connected to the first arm (110).

3. The robotic drive system of claim 2, wherein the first arm (110) and the second arm (114) are coupled allowing relative movement between the first arm (110) and the second arm (114) only along their respective longitudinal axes.

4. The robotic drive system of claim 3, further comprising a first flexible support (118) having a first distal end (120) and a first proximal end (122), the first distal end (120) being removably secured to the first cassette (112) and the first proximal end (122) being secured to a proximal end of the first arm (110), wherein a portion of the first flexible support (118), intermediate the first distal end (120) and the first proximal end (122), is positioned within the second cassette (116).

5. The robotic drive system of claim 3, wherein the sterile cassette assembly (100) further comprising a third cassette (126) and wherein the coupler (104) further comprises a third arm (128) being slidably connected to the second arm (114), the third cassette (126) connected to the third arm (128).

6. The robotic drive system of claim 5, wherein the second arm (114) comprises a first portion slidably engaged with the first arm (110) and a second portion slidably engaged with the third arm (128).

7. The robotic drive system of claim 5, wherein the sterile cassette assembly (100) further comprises a fourth cassette (136) and wherein the coupler (104) further comprises having a fourth arm (138) being slidably connected to the third arm (128), the fourth cassette (136) connected to the fourth arm (138).

8. The robotic drive system of claim 5, further comprising a second flexible support (130) having a second distal end (132) and second proximal end (134), the second distal end (132) of the second flexible support (130) being removably secured to the second cassette (116) and the second proximal end (134) being secured to a proximal end of the second arm (114), wherein a portion of the second flexible support (130) intermediate the second distal end (132) and the second proximal end (134) is positioned within the third cassette (126).

9. The robotic drive system of claim 3, further comprising an initial flexible support (146) having an initial distal end (150) and an initial proximal end (148), the initial distal end (150) being removably secured to a distal sheath connector (152) distal the first cassette (112) and the initial proximal end (148) being secured to a robotic drive housing, wherein a portion of the initial flexible support (146) intermediate the initial distal end (150) and the initial proximal end (148) is positioned within the first cassette (112).

10. The robotic drive system of claim 9, wherein the first arm (110) comprises a first guide (156) operatively guiding a portion of the initial flexible support (146) between the distal sheath connector (152) and a support anchor (154) on a housing of the robotic drive.

11. The robotic drive system of claim 4, wherein the second arm (114) comprises a second guide (158) operatively guiding a portion of the first flexible support (118) between the first cassette (112) and the proximal end of the first flexible support (118).

12. The robotic drive system of claim 8, wherein the third arm (128) comprises a third guide (162) operatively guiding a portion the second flexible support (130) between the second cassette (116) and the proximal end of the first flexible support (118).

13. The robotic drive of one of claims 1 to 12, wherein each cassette comprises a portion (222) that rests on a surface of a corresponding drive module during attachment of each cassette to the corresponding drive module.

14. The robotic drive of one of claims 1 to 13, wherein the first cassette (112) comprises a latch (210) releasably engaging a tab (190) in the drive module.

15. The robotic drive of one of claims 1 to 14, wherein the first cassette (112) comprises a cylindrical cavity receiving a cylindrical member of the drive module.

16. A robotic drive system for driving one or more elongated medical devices comprising:
a robotic drive comprising;
a first drive module (111); and
a second drive module (115) proximal to the first drive module (111);
a third drive module (125) proximal the second drive module (115), wherein each drive module moves independently along a longitudinal axis of the robotic drive; and
a sterile cassette assembly (100) comprising:
a first cassette (112) having a first arm (110);
a second cassette (116) having a second arm (114),
a third cassette (126) having a third arm (128),
a first flexible support (118) having a distal end and a proximal end attached to the first cassette (112), wherein a portion of the first flexible support (118) extends through the second cassette (116); and
a second flexible support (130) having a distal end and a proximal end attached to the second cassette (116), wherein a portion of the second
flexible support (130) extends through the third cassette (126);
wherein the first cassette (112), second cassette (116) and third cassette (126) are movable independent of one another, and wherein the first arm (110), second arm (114) and third arm (128) are slidably connected to one another.

17. The robotic drive system of claim 16, wherein the second arm (114) comprises a second guide (158) operatively guiding a portion of the first flexible support (118) between the first cassette (112) and a connector on the proximal end of the first arm (110).

18. The robotic drive system of claim 17, further comprising an initial flexible support (146) having a distal end secured to a sheath connector and a proximal end secured to a housing of the robotic drive, wherein a portion of the initial flexible support (146) moves through a channel in the first cassette (112).

19. The robotic drive system of one of claims 16 to 18, wherein the cassette assembly (100) can balance on a portion of the drive modules in an unlocked position.

20. The robotic drive system of claim 19, wherein each cassette comprises a latch (210) that releasably locks each cassette to a corresponding drive module.\

21. A sterile cassette assembly (100) comprising:
a first cassette (112),
a second cassette (116),
a third cassette (126), and
a coupler (104) coupling the first cassette (112), the second cassette (116), and the third cassette (126) together, wherein the first cassette (112), the second cassette (116) and the third cassette (126) are movable independent of one another toward and away from one another while coupled together with the coupler (104).

22. The sterile cassette assembly (100) of claim 21, further including a third cassette (126), wherein the coupler (104) couples the first cassette (112), the second cassette (116), and the third cassette (126) together, wherein the first cassette (112), the second cassette (116) and the third cassette (126) are movable independent of one another toward and away from one another while coupled together with the coupler (104).

23. The sterile cassette assembly (100) of claim 22, the coupler (104) comprising:
a first arm (110) secured to the first cassette (112),
a second arm (114) secured to the second cassette (116), and
a third arm (128) secured to the third cassette (126);
wherein the first arm (110), second arm (114) and third arm (128) are slidably connected to one another.

24. The sterile cassette assembly (100) of claim 23 further including:
a first flexible support (118) having a distal end and a proximal end being attached to the first cassette (112), wherein a portion of the first flexible support (118) extends through the second cassette (116); and
a second flexible support (130) having a distal end and a proximal (124) end attached to the second cassette (116), wherein a portion of the second flexible support (130) extends through the third cassette (126).
